# EUROPEAN PATENT APPLICATION

(11) **EP 3 498 216 A1**
(43) Date of publication of application: **19.06.2019**
(21) Application number: 17207150.8
(22) Date of filing: 13.12.2017
(51) Int. Cl.: A61B 90/30, A61B 90/35, G02B 6/26

(54) **SURGICAL LIGHT DEVICE AND LIGHTHEAD FOR SURGICAL LIGHT DEVICE**

(71) Applicant: Ondal Medical Systems GmbH, 36088 Hünfeld (DE)
(72) Inventor: Walters, Chris, Sandston, VA Virginia 23150 (US)
(74) Representative: Graf von Stosch Patentanwaltsgesellschaft mbH

(57) **Abstract**

The present application is directed to a surgical device, comprising a light source, and an illumination unit comprising a lighthead that is connected to a mount for mounting the illumination unit to a supporting structure by one or more support arms that are pivotally connected through joint units, wherein the light source is disposed remotely from the lighthead, in particular not mounted on the lighthead, and is coupled to the lighthead via light conductors. In addition, a lighthead for a surgical light device is disclosed, comprising an optical connector that is configured to be connectable to a light conductor that is connected to a light source.

## Description

The present invention relates to a surgical light device and a lighthead for a surgical light device.

Surgical light devices are used in medical environments such as operation theatres, as well as in treatment rooms in medical practices to provide localized illumination at a high intensity on a patient during an operation or treatment. For this, the known surgical light devices comprise a light source providing illumination at a high intensity that is suspended from a support structure such as a ceiling or a wall via a pivotable support system in order to adjust the position of the light source relative to the patient during the operation or treatment.

An example for a known surgical light device is described in EP 2 760 257 A1. It comprises a lighthead with a lighting arrangement comprising light emitting diodes (LEDs) which is connected by way of a deflectable spring arm to an operating theatre ceiling.

The known surgical light devices exhibit a variety of disadvantages. The equipment mounted in the lighthead, including the light sources and corresponding power supply, as well as accompanying optical elements are costly. Furthermore, the equipment installed in the lighthead yields a high weight of the lighthead, so that the handling of the surgical light device becomes burdensome. The larger weight of the known lightheads also requires the support system to provide sufficient sturdiness, so that the materials used for the support system usually require metal constructions.

The electronics installed in the lighthead generate magnetic fields. In combination with the metal construction of the support system, the known surgical light devices are rendered ill-suited for the usage with applications that are sensitive to magnetic field disturbances, such as magnetic resonance therapy or the like. In addition, if the light source and its accompanying power source are to be upgraded or replaced, the configuration of the lighthead has to be modified or the lighthead has to be replaced, which yields an increased constructional effort. Furthermore, the electronics mounted in the lighthead act as heat source, which is undesirable in an operating environment.

In light of the above, it is an object of the present invention to provide illumination means that allows to overcome the disadvantages of known surgical light devices described above.

The above object is solved by a surgical light device according to the subject-matter of claim 1 and by a lighthead according to the subject-matter of claim 9. Preferred embodiments of the invention are indicated by the subject-matter of the dependent claims.

Specifically, the present invention provides a surgical light device comprising a light source and an illumination unit comprising a lighthead that is connected to a mount for the mounting illumination unit to a supporting structure by one or more support arms that are pivotally connected through joint units, wherein the light source is disposed remote from the lighthead, in particular not mounted on the lighthead, and is coupled to the lighthead via light conductors. Accordingly, the invention provides a surgical light device comprising a light source and an illumination unit, a mount for mounting the illumination unit and at least one supporting structure, wherein the supporting structure comprises one or more support arms that are pivotally connected through joint units. For the surgical light device, the light source is physically separate from the illumination unit comprising the lighthead. The illumination unit comprising the lighthead is disposed preferably at the distal end of the most distal support arm. The light source is preferably arranged within the support section mounted to the mount or within the most proximal support arm.

It is a core aspect of the present invention that the light source (including its power supply) is physically and spatially separated from the lighthead. The light emitted from the light source is coupled into a light conductor that is coupled to the lighthead. Light conductors are assemblies similar to an electrical cable, but used to carry light. As light conductors, fiber optic cables or fiber optic bundles may be used. Optical fibers comprise a transparent core and a cladding layer surrounding the core, wherein the difference in refractive index between the two is chosen such that light travelling in the core is subject to total internal reflection at the interface between the core and the cladding. Light conductors provide a reliable optical connection between the light source and the lighthead at low cost.

It should be noted that the light conductors used in the context of this invention for the transport of light from the light source to the light head are required to withstand a high light intensity without damage. Light conductors that are typically used for the transmission of data signals are not suitable in this context, since their core diameter usually is smaller than 1 mm. Small core diameters are required for the achievement of high data rates, but are not suitable to transmit light of high intensity. Therefore, the light conductors used in the surgical light device according to this invention typically have a core diameter of more than 1 mm, preferably a core diameter of 0.5 cm or more or of 1 cm or more.

The light source may be a LED light source, preferably a high-power LED source or a conventional halogen light source that is equipped with suitable optical components to couple the emitted light into the light conductors.

The equipment mounted in the lighthead can thus be reduced to optical components that are required to manipulate the light intensity distribution in a suitable manner. Thereby, it is possible to design low-cost lightheads.

Furthermore, spatial separation of the light source from the lighthead allows for drastic reduction of the weight of the lighthead. The invention facilitates the handling of the illumination unit when position and orientation of the lighthead are adjusted. Moreover, the reduced weight of the lighthead alleviates the requirements for the stability of the support arms and the mount, so that lighter and cheaper materials can be used for these elements. Altogether, the construction of the entire illumination unit comprising the lighthead, the support arms, the joint units and the mount may be envisaged by cheaper and lighter materials.

Since no electronics are required on the lighthead, virtually no heat is generated in the vicinity of the lighthead. Also, generation of stray magnetic fields in the lighthead is avoided, such that the application of the surgical light device in magnetically sensitive fields of applications, such as magnetic resonance therapy or imaging, is enabled.

Beyond that, physical separation of the light source from the lighthead greatly simplifies the maintenance or replacement of the light source, since no technical modifications of the sensitive lighthead have to be performed. The spatially separated provision of the light source positioned remotely from the lighthead also allows for operating multiple light sources in parallel, that are easily swapped by coupling different light sources to the lighthead via the light conductors. Thereby, reliability of the surgical light device is enhanced. It is also possible to use high power light sources that are not usable on conventional lightheads due to limitations with regard to size and weight.

The support structure to which the illumination unit is mountable may be a wall or a ceiling. The number of the support arms that pivotally connect the lighthead to the mount is not particularly limited. The surgical light device may comprise one support arm that is pivotally connected to the mount with one end, and pivotally connected to the lighthead with the other end. For increased flexibility, two or more (e.g. 3 or 4) support arms may be provided in a chain-like manner, with a first support arm being pivotally connected to the support with one end, and the most distant (distal) support arm being pivotally connected to the light source with one end, and the interposed support arms being pivotally connected to each other. The joint units that provide the pivotable connections may be realized by hinge joints which allow a pivoting of connected support arms only in a common plane, or by rotational joints, where the support arms are arranged in two essentially parallel planes and are rotatable along a rotation axis that is perpendicular to both planes. The joint units may also be realized by ball joints. It is conceivable to use only one type of joint unit in the medical light device, or to use different types of joint units for different pivotable connections in the medical light device.

By a preferred embodiment, the light source is provided as a component being completely separate from the illumination unit. Since the light generated by the light source is transmitted to the lighthead via the light conductors without significant losses, no limitations are imposed on the placement of the light source relative to the lighthead. The light source may, for example, be installed on the outside wall of a building, or in the same room as the surgical light device, or in a room adjacent thereto. Such an embodiment enables to further reduce the impact of heat and stray magnetic signals generated by the light source on the vicinity of the lighthead.

In another preferred embodiment, the light source is positioned inside one of the support arms or within the mount. Such an embodiment allows for a compact design of the surgical light device while providing sufficient spatial separation between the light source and the lighthead.

According to a preferred embodiment of the invention, the light conductors are arranged inside the support arms. By arranging the light conductors inside the support arms, the handling of the surgical light device can be improved, since the light conductors are not interfering with the movement of the support arms.

It is further preferred that light couplers for transmitting light between the support arms are arranged inside the joint units. Such light couplers are advantageous in order to transfer the light from one support arm to an adjacent, pivotally connected support arm. If the joint units are hinge joints that allow pivoting of the connected support arms only in a common plane, or ball joints, the light couplers may simply be constituted by a section of the light conductor that is disposed inside the joint units, or the light conductor may pass through the joint unit. If the joint units are rotational joints, the light couplers may be realized by fiber optic rotary joints that provide a freely rotatable connection without generating torsional stress on the light conductor. The fiber optic rotary joints are preferably configured to be connectable to light conductors with a core diameter of 1 mm or more, preferably of 0.5 cm or more or more preferably with a core diameter of 1 cm or more.

It is further preferred that the illumination unit, particularly the lighthead and/or the support arms and/or the joint units and/or the mount, is/are composed of non-magnetic material. Since the weight of the lighthead is reduced by providing the light source separately therefrom, the requirements on the illumination unit with respect to its mechanical strength are significantly reduced, so that non-magnetic, light materials like plastic or the like may be used. Hereby, interference properties of the surgical light device with respect to magnetically sensitive environments are further reduced.

It is also preferable that the lighthead comprises a light distributor for distributing light transmitted from the light source via the light conductors. The light distributor is optically coupled to an outer end of the light conductors that provides the light on the lighthead. Hereby, the light transmitted from the light source via the light conductors may be distributed over a predetermined area in order to achieve the desired illumination properties of the lighthead. The lighthead may further comprise optical elements to modify the light distribution of the light emitted from the lighthead, e.g., to focus the light to a predetermined spot size.

It is further preferable that the lighthead is configured to be detachable from the support arm. Preferably, the lighthead comprises an optical connector that is configured to be connectable to a light conductor that is connected to the light source. The light conductor that is connectable to the optical connector of the lighthead may be arranged in the support arm. Such a feature enables an easy replacement of the lighthead.

The object of the invention is also solved by a lighthead for a surgical light device comprising an optical connector that is configured to be connectable to a light conductor that is connected to a light source. By such a configuration, the light source does not have to be provided on the lighthead itself either. Rather, light generated by a light source that is provided separately from the lighthead may be coupled into the lighthead. Thus, a lighthead with low weight and without electrical components may be provided.

It is preferred that the lighthead is attachable to and detachable from the support arm. Such an embodiment enables a mounting of the lighthead on a support structure that is commonly used for surgical light devices.

Preferably, the lighthead comprises a light distributor for distributing light transmitted to the lighthead via the optical connector. The light distributor is optically connected to the optical connector. This allows to distribute the light transmitted to the lighthead over a larger area, so that a larger area can be illuminated with constant intensity.

The above and further features and advantages of the invention will become more readily apparent from the following detailed description of preferred embodiments of the invention with reference to the accompanying drawings, in which like reference signs designate like features, and in which:
Fig. 1 is a schematic illustration of a surgical light device according to an embodiment of the present invention;
Fig. 2 is a schematic illustration of a surgical light device according to another embodiment of the present invention;
Fig. 3 is a schematic illustration of a surgical light device according to yet another embodiment of the present invention;
Fig. 4 is a schematic cross-sectional view of a joint unit that is configured as a hinge joint;
Fig. 5 is a schematic view of a lighthead according to the present invention.

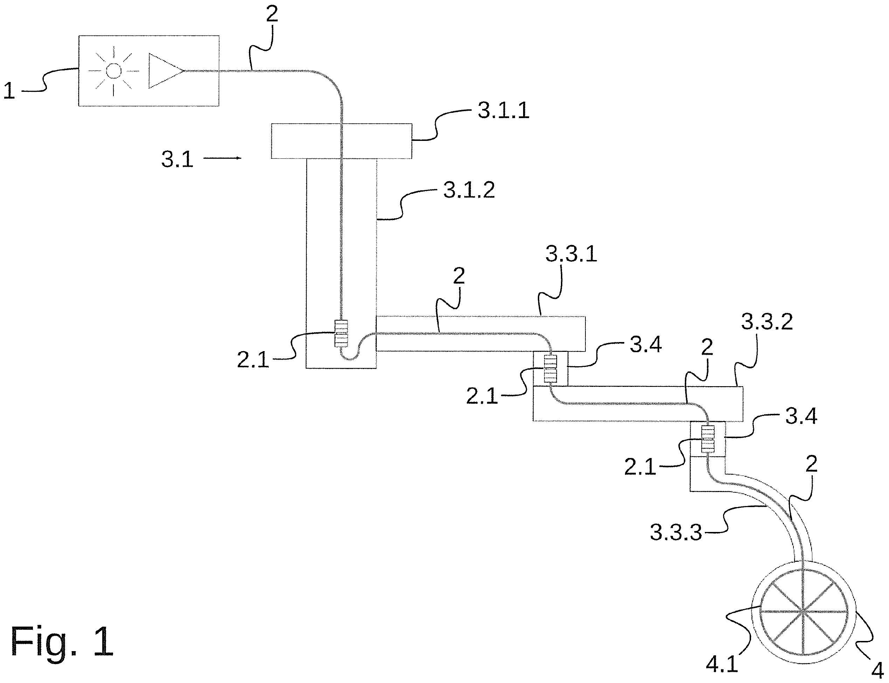

Fig. 1 is a schematic illustration of a surgical light device according to an embodiment of the present invention. The surgical light device comprises a light source 1 and an illuminating unit. The illuminating unit comprises a mount 3.1, support arms, joint units 3.4 and a lighthead 4. In Fig. 1, three support arms 3.3.1, 3.3.2 and 3.3.3 are shown, the entirety of which is denoted by 3.3 in the following.

The mount 3.1 comprises a mounting section 3.1.1 for attachment to a support structure and a support section 3.1.2 for connecting the mount 3.1 to support arm 3.3.1. Support section 3.1.2 may be pivotally attached to the mount 3.1 such that it may rotate or, alternatively, it may be fixed to the mount 3.1. The mount 3.1 shown in Fig. 1 is configured as a ceiling mount for mounting the illuminating unit to a ceiling. It is also possible to configure the mount 3.1 as a wall mount, with the support section 3.1.2 extending horizontally from the mounting section 3.1.1. It is further possible to configure the mount 3.1 as a clampable mount with a mounting section 3.1.1 that comprises a clamp mechanism that is clampable to a sheet-like support structure or a rod-like structure.

The support arm 3.3.1 is pivotally attached to the support section 3.1.2 by a rotational joint (not shown) that allows the support arm 3.3.1 to be rotated around an axis defined by the support section 3.1.2. The support arm 3.3.1 that is attached to the support section 3.1.2 with one end is connected to a further support arm 3.3.2 at its other end via a joint unit 3.4. The support arm 3.3.2 is connected to a further support arm 3.3.3 by a further joint unit 3.4. The support arm 3.3.3 is connected to the lighthead 4. The connection between support arm 3.3.3 and the lighthead 4 may be rigid or, alternatively, be constituted by a further joint unit. The support arm 3.3.3 has a bent shape that facilitates adjusting the position and orientation of the lighthead 4.

The light source 1 comprises a power supply (not shown) and generates light that is coupled into a light conductor 2. The light conductor 2 is connected to the light source 1 via a suitable connector. The light conductor 2 may be realized by a fiber optic cable or by a fiber optic bundle composed of a plurality of optical fibers. As connector, a conventional fiber optical coupler may be employed. The light coupled into the light conductor 2 is transmitted through the light conductor 2 to the illumination unit.

In the illumination unit, an optical path is established by a continuous connection of light conductors 2 that is guided to the lighthead 4. The light conductors 2 are arranged inside the support arms 3.3 that are configured as hollow components. In the lighthead 4, the light is coupled into an optical light distributor 4.1 that is adapted to distribute the light over an extended area in the lighthead 4 so that an even illumination pattern can be achieved over a sufficiently large area.

The light conductors 2 in the individual support arms are connected by light couplers 2.1. The light couplers 2.1 provide an optical connection between the individual light conductors 2. The joint units 3.4 shown in Fig. 1 are depicted as rotation joints that enable pivoting motion of the connected support arms in two mutually parallel planes. In the joint units 3.4 shown in Fig. 1, the optical connection lies on the axis of rotation of the joint unit 3.4.

A pivoting motion of the support arms 3.3 around the axis of rotation of the joint unit 3.4 induces torsional strain in the optical connection, since the optical connection is positioned on the axis of rotation of the joint unit 3.4. If a section of light conductor 2 itself were used as optical connection in the rotation joint 3.4, the torsional strain would potentially lead to damage or breakage of the light conductor. For this reason, the light couplers 2.1 are provided as optical connection inside the joint units 3.4. The light couplers 2.1 are preferably configured as fiber optic rotary joints that provide an optical connection between the light conductors 2 in adjacent support arms 3.3 that remains unaffected by pivoting motions of the support arms 3.3.

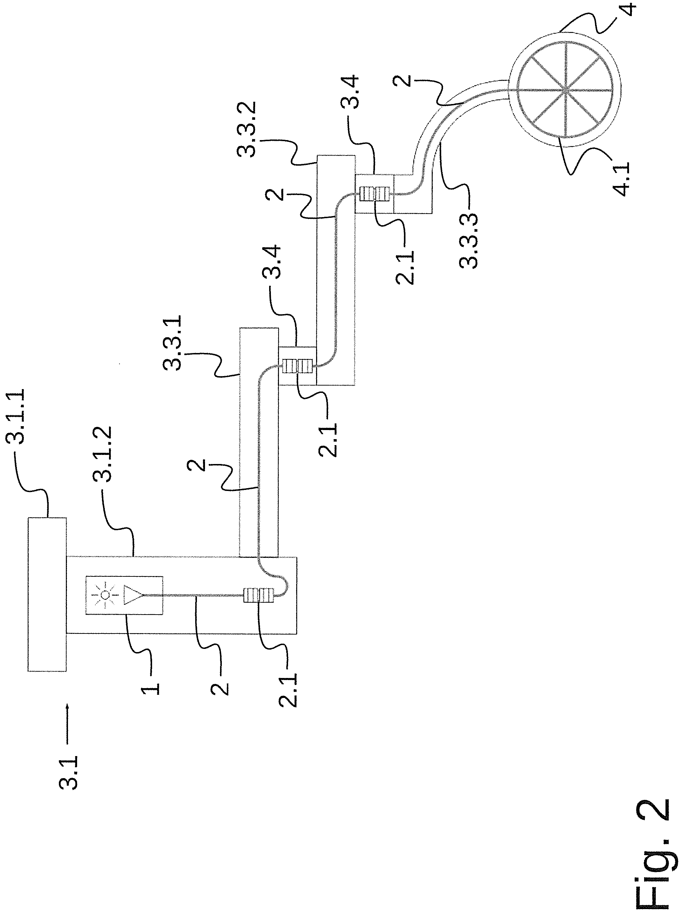

Fig. 2 shows an alternative embodiment of the surgical light unit according to the present invention. The configuration of the illumination unit of Fig. 2 is essentially identical to that of Fig. 1. However, in the embodiment according to Fig. 2, the light source 1 is positioned inside the mount 3.1.

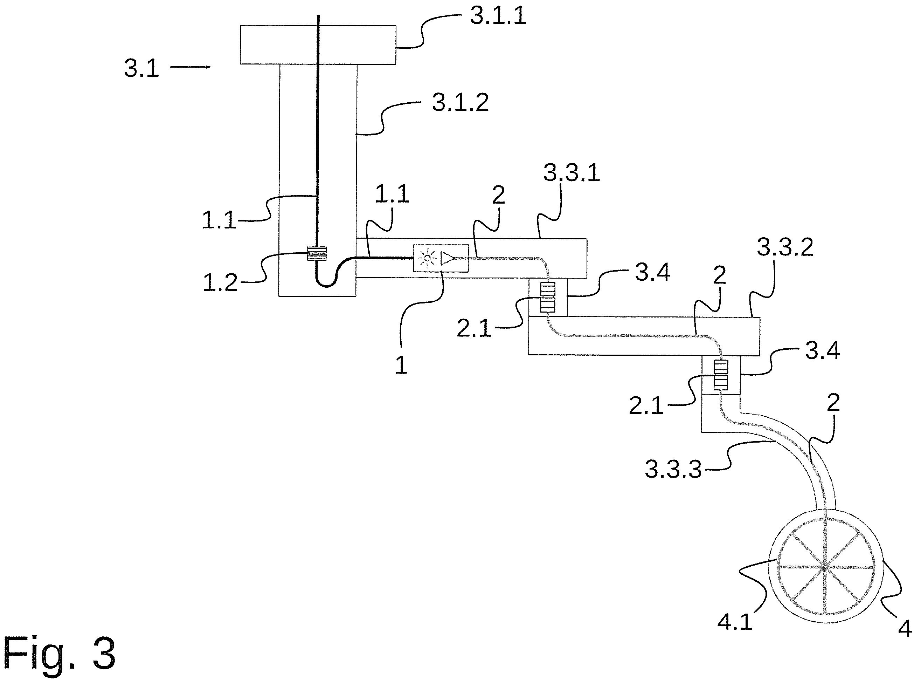

Fig. 3 shows a further embodiment of the surgical light device according of the present invention. The configuration of the illumination unit of Fig. 3 strongly resembles that of Fig. 1 and 2. However, in the embodiment according to Fig. 3, the light source 1 is positioned inside a support arm 3.3. As shown in Fig. 3, the light source 1 is preferably positioned inside the first (proximal) support arm 3.3.1 that is directly connected to the mount 3.1. By such a configuration, sufficient distance between the light source 1 and the lighthead 4 is still maintained.

The embodiment according to Fig. 3 bears the advantage that the light source 1 is installed in the support arm structure that the corresponding light head 4 is connected to. By such a configuration, multiple support arm structures, e.g. 2 or 3 support arm structures, may be mounted to the mount 3.1, each support arm structure carrying an individual light source 1 or at least one of them carrying light source 1. This allows for a highly flexible and modular surgical lighting arrangement.

According to the embodiment of Fig. 3 the power source 1 has to be connected to an electrical power source. For this, a power line 1.1 is provided in the support 3.1. Since the first support arm 3.3.1 in which the light source 1 is installed is pivotally attached to the support section 3.1.2, an electrical sliding contact 1.2 is provided, in order to establish a rotatable electrical connection that is unaffected by pivoting motions of the support arm 3.3.1. The electrical sliding contact 1.2 may be configured as a slip ring.

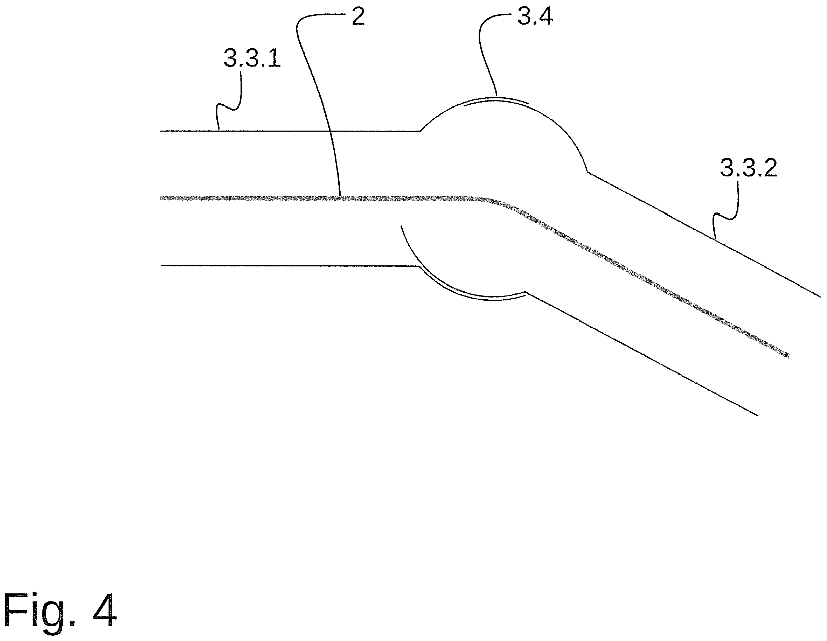

Joint units 3.4 shown in Fig. 1 to 3 are rotational joints. One or more joint units 3.4 of the illumination unit may also be replaced by hinge joints or ball joints. In this case, the light couplers 2.1 may be omitted. Fig. 4 shows a schematic cross-sectional view of a joint unit 3.4 that connects two support arms 3.3.1 and 3.3.2 and is configured as a hinge joint. The hinge joint comprises an opening inside the joint unit 3.4 through which the light conductor 2 can be guided. Since the light conductor 2 is flexible and the hinge joint only allows a pivoting motion of the support arms 3.3.1 and 3.3.2 in a common plane that does not induce a torsional strain in the light conductor 2, the light coupler 2.1 used in the rotational joints of Fig. 1 to 3 may be omitted. The optical connection between the two adjacent support arms 3.3.1 and 3.3.2 is established by a continuous light conductor 2 in this case. The same optical connection can be employed if the joint unit 3.4 is established by a ball joint.

In the embodiments described above, the surgical light device comprises one light source 1 that is positioned remotely from the lighthead 4 and coupled thereto by one optical path of serially connected light conductors 2. The surgical light device according to the present invention may also comprise a plurality of light sources 1, and/or a plurality of optical paths. The light emitted from multiple light sources may be coupled into a common optical path, or individual light sources may be provided with separate optical light sources that are guided to lighthead 4, respectively. Such configurations allow to increase the light intensity available in the lighthead 4.

It is also possible to provide light of various colors of the visible spectrum at the lighthead 4 by providing light sources that emit light of defined wavelengths. Light of distinct colors emitted from different light sources may be transmitted to the lighthead 4 via separate light conductors 2 or may be merged by coupling it into a common light conductor 2. By each of these configurations it is possible to provide a surgical light device with a lighthead 4 of low weight that requires no electronic components (but only passive components), which is suitable for use in a magnetically sensitive environment whilst providing light with high intensity of whatever desired color.

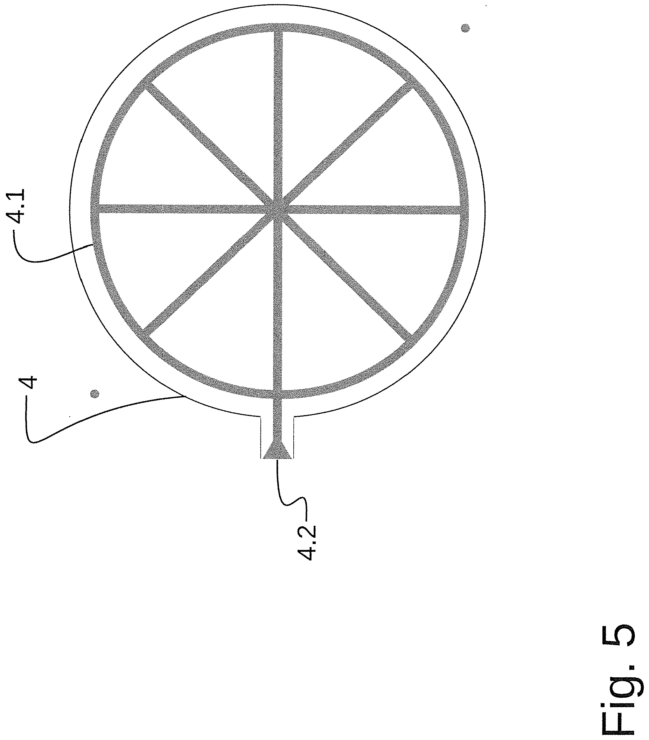

The connection between the lighthead 4 and its adjacent support arm 3.3.3 may be configured such that the lighthead 4 is detachable from the support arm 3.3.3. Fig. 5 is a schematic view of a lighthead 4 according to the present invention. The lighthead 4 comprises an optical connector 4.2 that may be realized by an optical coupler connectable to an output coupler provided on a light conductor 2 in the support arm 3.3.3. If multiple optical paths are provided in the illumination unit, the lighthead 4 may comprise the respective number of optical connectors 4.2 for connecting the lighthead 4 to multiple light sources.

The optical light distributor 4.1 that is configured to distribute the light transmitted to the lighthead via the optical connector 4.2 is illustrated as a circle with four straight connections in Fig. 5, but may have an arbitrary shape on the lighthead 4. By varying the shape of the light distributor 4.1, the light emission pattern of the lighthead 4 can be modified according to individual requirements.

The lighthead 4 may further comprise optical components (not shown) such as lenses and the like that may be used to manipulate the radiation pattern of the light emitted from the lighthead 4. The surface of the lighthead 4 on which the optical light distributor 4.1 is disposed may further comprise reflective material or whatever coating in order to increase the intensity of the emitted light.

### Reference Sign List

- 1: light source
- 1.1: power line
- 1.2: electrical sliding contact

- 2: light conductor
- 2.1: light coupler

- 3.1: mount
- 3.1.1: mounting section
- 3.1.2: support section
- 3.3; 3.3.1, 3.3.2, 3.3.3: support arm
- 3.4: joint unit

- 4: lighthead
- 4.1: optical light distributor
- 4.2: optical connector

## Claims

1. Surgical light device, comprising
a light source (1), and
an illumination unit comprising a lighthead (4) that is connected to a mount (3.1) for mounting the illumination unit to a supporting structure by one or more support arms (3.3; 3.3.1, 3.3.2, 3.3.3) that are pivotally connected through joint units (3.4), wherein the light source (1) is disposed remotely from the lighthead (4), in particular not mounted on the lighthead (4), and is coupled to the lighthead (4) via light conductors (2).

2. Surgical light device according to claim 1,
wherein the light source (1) is provided physically separately from the illumination unit.

3. Surgical device according to claim 1,
wherein the light source (1) is positioned inside one of the support arms (3.3) or within the mount (3.1).

4. Surgical light device according to one of the preceding claims,
wherein the optical conductors (2) are arranged inside the support arms (3.3; 3.3.1, 3.3.2, 3.3.3).

5. Surgical light device according to one of the preceding claims,
wherein light couplers (2.1) for transmitting light between the support arms (3.3; 3.3.1, 3.3.2, 3.3.3) are arranged inside one or more of the joint units (3.4).

6. Surgical light device according to one of the preceding claims,
wherein the illumination unit, particularly the lighthead (4) and/or the support arms (3.3; 3.3.1, 3.3.2, 3.3.3) and/or the joint units (3.4) and/or the mount (3.1), is/are composed of a non-magnetic material.

7. Surgical light device according to one of the preceding claims,
wherein the lighthead (4) comprises a light distributor (4.1) for distributing light transmitted from the light source (1) via the optical conductors (2).

8. Surgical light device according to one of the preceding claims,
wherein the lighthead (4) is configured to be detachable from the support arm (3.3.3) and comprises an optical connector (4.2) that is configured to be connectable to a light conductor (2) that is connected to the light source (1).

9. Lighthead (4) for a surgical light device, comprising an optical connector (4.2) that is configured to be connectable to a light conductor (2) that is connected to a light source (1).

10. Lighthead (4) for a surgical light device according to claim 9, wherein the lighthead (4) is attachable to and detachable from support arm (3.3), preferably from the most distant support arm.

11. Lighthead (4) for a surgical light device according to claim 9, comprising a light distributor (4.1) for distributing light transmitted to the lighthead via the optical connector (4.2).
